(19) Europäisches Patentamt
European Patent Office
Office européen des brevets

(11) Publication number: 0 089 703 B1

# (12) EUROPEAN PATENT SPECIFICATION

(45) Date of publication of patent specification: 22.10.86

(51) Int. Cl.[4]: C 07 D 249/08, A 01 N 43/653 // C07C149/16, C07C149/14, C07C149/34

(21) Application number: 83200278.6

(22) Date of filing: 23.02.83

## (54) Biologically active triazolyl derivatives.

(30) Priority: 11.03.82 GB 8207143

(43) Date of publication of application: 28.09.83 Bulletin 83/39

(45) Publication of the grant of the patent: 22.10.86 Bulletin 86/43

(84) Designated Contracting States: AT BE CH DE FR GB IT LI LU NL SE

(56) References cited:
EP-A-0 027 177
EP-A-0 056 860

(73) Proprietor: SHELL INTERNATIONALE RESEARCH MAATSCHAPPIJ B.V.
Carel van Bylandtlaan 30
NL-2596 HR Den Haag (NL)

(72) Inventor: Ten Haken, Pieter
Konkelboet The Street
Eastling Nr. Faversham Kent (GB)
Inventor: Appleton, Robert Frederick
30 Westerham Road
Sittingbourne Kent (GB)
Inventor: Webb, Shirley Beatrice
17 North Street Ashford Road
Sheldwich Faversham Kent (GB)

(74) Representative: Hunter, Keith Roger Ian et al
4 York Road
London SE1 7NA (GB)

EP 0 089 703 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to biologically active triazolyl derivatives.

Certain fungicidal triazole derivatives, for example 1-trityl-1,2,4-triazole, are described in Swiss Patent Specification 488,713.

EP—A—27177 discloses a process for preparing certain compounds described as having fungicidal activity, which compounds include triazolyl derivatives of formula:

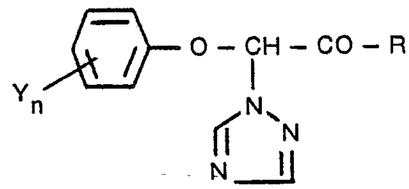

wherein R is alkyl or optionally substituted phenyl, Y is halogen, alkyl, alkoxy, cycloalkyl, nitro, the group —$COOR^1$ (wherein $R^1$ is alkyl or optionally substituted phenyl or benzyl), optionally substituted phenyl or optionally substituted phenoxy and n is 0 to 4.

EP—A—56860 discloses a class of fungicidal sulphonate derivatives, which derivatives include triazolyl derivatives of formula

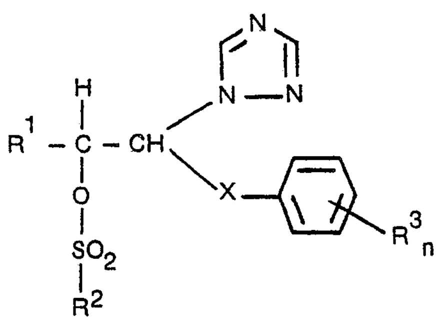

wherein $R^1$ and $R^2$ are independently selected from $C_{1-6}$ alkyl, (optionally substituted by fluorine, chlorine, bromine or iodine), $C_{1-4}$ alkoxy and phenyl (optionally substituted by fluorine, chlorine, bromine, cyano, $C_{1-6}$ alkyl, $C_{1-4}$ alkoxy, phenyl or phenoxy), $R^3$ is hydrogen, halogen, $C_{1-6}$ alkyl, $C_{1-4}$ alkoxy, cyano, phenyl or phenoxy (phenyl or phenoxy being optionally substituted by halogen or $C_{1-6}$ alkyl), n is 1 to 5 and X is O or S. These derivatives are prepared from the corresponding hydroxy intermediates of formula:

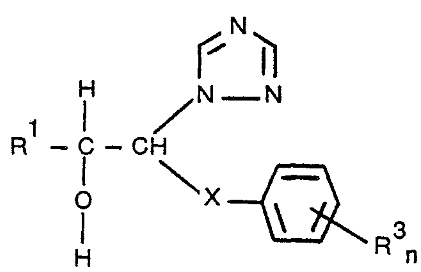

The hydroxy intermediate of the above formula wherein $R^4$ is t-butyl, X is 0 and $R^3_n$ is 4-chloro is 1-(4-chlorophenoxy)-3,3-dimethyl-1-(1,2,4-triazol-1-yl)-butan-2-ol, the known fungicide triadimenol.

It has now been found that a particular class of triazolyl compounds, part of which comprises novel compounds, possesses useful biological activity.

According to the present invention there are provided fungicidal and plant growth regulating compositions which comprise a carrier and, in association therewith, a compound of the general formula

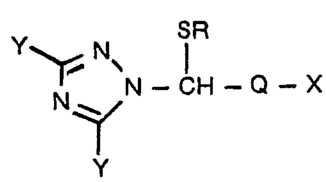

(I)

or an acid addition salt or metal salt complex thereof, wherein X represents a phenyl group which is

unsubstituted or substituted by up to 3 of the same or different substituents selected from halogen atoms and cyano, nitro, methylenedioxy, $C_{1-6}$ alkylsulphonyl, $C_{1-6}$ alkyl, $C_{1-6}$ haloalkyl, $C_{2-6}$ alkenyl, $C_{2-6}$ alkynyl, $C_{1-6}$ alkoxy, $C_{1-6}$ haloalkoxy, $C_{1-6}$ alkylthio, ($C_{1-6}$ alkoxy)carbonyl, phenyl and phenoxy groups; each Y independently represents a hydrogen or halogen atom or a $C_{1-6}$ alkyl group; Q represents a —CO—, —CS—, —CH.OH— or —CH.SH— group; and R represents a hydrogen atom, an alkyl group having up to 12 carbon atoms, which is unsubstituted or substituted by one or more substituents selected from halogen atoms, $C_{1-6}$ alkoxy, $C_{1-6}$ alkylthio, cyano, carboxy, ($C_{1-6}$ alkoxy)carbonyl, hydroxy, amino, $C_{3-7}$ cycloalkyl, phenyl and phenoxy groups, a $C_{3-7}$ cycloalkyl group which is unsubstituted or substituted by one or more substituents selected from halogen atoms and $C_{1-6}$ alkyl, $C_{1-6}$ alkoxy and $C_{1-6}$ alkylthio groups, or a phenyl group which is unsubstituted or substituted by up to 3 of the same or different substituents selected from halogen atoms and cyano, nitro, methylenedioxy, $C_{1-6}$ alkylsulphonyl, $C_{1-6}$ alkyl, $C_{1-6}$ haloalkyl, $C_{2-6}$ alkenyl, $C_{2-6}$ alkynyl, $C_{1-6}$ alkoxy, $C_{1-6}$ haloalkoxy, $C_{1-6}$ alkylthio, ($C_{1-6}$ alkoxy)carbonyl, phenyl and phenoxy groups.

The invention also comprises compounds *per se* of formula I as defined above, provided that when X represents a phenyl group which is unsubstituted or substituted exclusively by one or more substituents selected from halogen atoms and cyano, $C_{1-6}$ alkyl, $C_{1-4}$ alkoxy, phenyl and phenoxy groups, each Y represents a hydrogen atom and R represents a phenyl group which is unsubstituted or substituted exclusivity by one or more substituents selected from halogen atoms and $C_{1-4}$ alkyl, $C_{1-4}$ alkoxy, cyano, phenyl and phenoxy groups, Q represents a —CO—, —CS—, or —CH.SH— group.

Throughout this Specification, unless otherwise stated, any alkyl, alkenyl or alkynyl group present preferably contains up to 4 carbon atoms.

The phenyl group X is preferably unsubstituted, monosubstituted or disubstituted. Preferred substituents are selected from bromine, chlorine and fluorine atoms, and nitro, cyano, alkyl, haloalkyl, alkoxy, haloalkoxy and alkylthio groups. Especially preferred substituents are halogen atoms, especially chlorine and fluorine atoms, and methyl, methoxy, trifluoromethyl, trifluoromethoxy, methylthio, cyano and nitro groups, with mono- and di-halophenyl groups X being most preferred.

Each Y preferably independently represents a methyl group or, especially, a hydrogen atom. Most preferably both Y's represent hydrogen atoms.

Preferably Q represents a —CO— or —CHOH— group.

An alkyl group represented by R has up to 12, preferably up to 6, carbon atoms, and such groups are preferably either unsubstituted or substituted by one or more halogen atoms. A phenyl group R may, for example, be substituted by the preferred substituents given above for the group X. Preferably R represents an unsubstituted alkyl group having up to 6 carbon atoms, especially an alkyl group having 3 or 4 carbon atoms.

The compounds of formula I form acid addition salts. Such salts may be with organic or inorganic acids. For example they may be with sulphonic acids, such as benzene- or toluene-sulphonic acids, carboxylic acids, such as oxalic or acetic acid, or mineral acids such as hydrohalic acids, particularly hydrochloric acid. The compounds of formula I also form complexes with metal salts, for example salts, for example halides, of calcium, copper, iron, zinc or manganese.

It will be appreciated that the 2-carbon atom of the ethane base unit is asymmetric and gives rise to isomeric forms of the compounds of the invention. Further isomeric forms of the compounds may also arise as a result of particular substituents. The invention encompasses all individual isomers as well as mixtures of isomers.

Compounds of formula I are conveniently prepared by a process which comprises reacting a 2-halo-1-phenylethane derivative of formula:

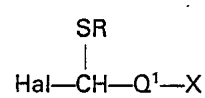

(II)

wherein X and R have the meanings given above, $Q^1$ is a —CO— or —CS— group, and Hal is a halogen atom, preferably a bromine atom, with a triazole derivative of formula:—

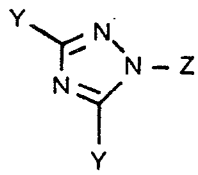

(III)

wherein Y has the meaning given above and Z represents a hydrogen atom or one equivalent of an alkali or alkaline earth metal; and if desired, converting the resulting compound of formula I into another compound of formula I. The process may be carried out in the absence of solvent, but preferably a solvent is present.

Polar organic solvents are especially suitable, for example, nitriles, such as propionitrile or acetonitrile; sulphoxides, such as dimethyl sulphoxide; amides, such as dimethyl acetamide or dimethyl formamide; ketones, such as acetone, methyl ethyl ketone, or diethyl ketone; ethers, such as diethyl ether, tetrahydrofuran or dioxan; nitroalkanes, such as nitromethane; and chlorinated hydrocarbons, such as methylene chloride or chloroform.

When Z is a hydrogen atom the reaction should desirably be carried out in the presence of an acid binding agent. This may conveniently be an appropriate excess of the triazole of formula III. However it is also possible to use other organic or inorganic acid binding agents conventionally used in such processes, such as amines, e.g., alkylamines, cycloalkylamines, aralkylamines, or aromatic amines, for example triethylamine, dimethylbenzylamine, dicyclohexylamine, pyridine or diazabicyclooctane; metal hydrides such as sodium hydride; alkali metal carbonates such as sodium or potassium carbonate; or metal hydroxides such as potassium hydroxide.

The reaction temperature may vary over a wide range, for example 30 to 180°C, especially 50 to 120°C. Generally the reaction may be carried out conveniently at reflux temperature.

The compound resulting from the reaction of the compound II with the compound III may be converted into any other desired compound of formula I by methods analogous to methods known in the art. For example, a —CO— or —CS-group Q may be reduced using any suitable reducing agent, to the corresponding —CH.OH— or —CH.SH— group. Suitable reducing agents include gaseous hydrogen over a catalyst, for example a palladium or platinum catalyst, and, especially hydride reducing agents. Complex metal hydrides and borohydrides such as sodium borohydride are especially suitable. A resulting free base of formula I may be converted into an acid addition salt or a metal salt complex by reaction with the desired acid or metal salt, or a resulting acid addition salt may be converted into the corresponding free base by reaction with an acid binding agent or base.

When preparing a compound of formula I in which Q is —CS— or —CH.SH—, it may be desirable first to prepare the corresponding compound in which Q is —CO—, and subsequently to replace the oxygen atom by a sulphur atom by known methods, for example by reaction with an inorganic sulphide such as $P_2S_5$.

The compounds of formula II may be prepared in a manner known in the art, for example by halogenation of a compound of formula:

$$RS—CH_2—Q^1—X \qquad (IV)$$

in which X, R and $Q^1$ have the meanings given above. Thus the compound of formula IV may be reacted with a halogenating agent, for example, $Br_2$, preferably in the presence of a solvent, and also an initiator such as light. The solvent may suitably be a halogenated hydrocarbon, for example, a haloalkane such as tetrachloromethane. The reaction suitably takes place at 0° to 60°C.

The compounds of formula III are known or can be made by methods known in the art, for example by reacting the 1,2,4-triazole with an alkali or alkaline earth metal hydride such as sodium hydride, suitably in an inert solvent such as dimethoxy ethane or pyridine.

The compounds of formula IV can be prepared from the corresponding 2-halo-1-phenylethane derivative by reaction with a compound of formula RSM, where R has the meaning given above and M is an alkali metal, for example, sodium or potassium. The reaction may be carried out in the presence of an inert solvent, for example acetone or dioxan and at temperatures of from 0° to 100°C. Preferably the 2-halo-1-phenylethane derivative is a 2-bromo or 2-chloro derivative.

Conveniently in many cases the compound of formula IV can be converted, without isolation of the intermediate halo compound of formula II, to the appropriate compound of formula I.

The compounds of formula I may be isolated from the reaction medium in the usual manner, and it will be appreciated that the differing isomeric forms can be isolated from one another in a manner known in the art, for example chromatography.

The invention also provides the use of a compound of formula I or composition according to the invention as a fungicide and/or a plant growth regulant. Further in accordance with the invention there is provided a method of combating fungal attack and/or regulating plant growth at a locus, by treating the locus with a compound of formula I or a composition according to the invention; particularly useful is a method of protecting crop plants from fungal attack comprising treating crop plants subject to or subjected to fungal attack, seeds of such crop plants or the medium in which such plants are growing or are to be grown with a compound of formula I or an acid addition salt or a metal salt complex thereof or a composition containing such a compound.

The active compound is generally applied at a dosage of from 0.025 to 4 kg/ha, depending upon the locus to be treated. In general, preferred rates for application as a fungicide are in the range 0.025 to 1.0 kg/ha. Especially preferred rates for application to plant foliage for prophylactic or therapeutic treatment against fungi, will be in the range 0.025 to 0.25 kg/ha; for soil treatment preferred rates of application may vary widely, for example 0.1 to 1.0 kg/ha; in use for seed treatment, preferred rates of application may be 0.05 to 1.0 g/kg. For use as a plant growth regulant, preferred rates of application are generally higher, for example 0.25 to 2.5 kg/ha.

When used as fungicides, compounds of the general formula I and salts and complexes thereof in

which Q is a —CO— group are in general preferred, whereas compounds in which Q is a —CH.OH— group may be preferred for use as plant growth regulants. Fungal diseases controlled include particularly the powdery mildews. Plant growth regulant effects include the depression of growth which can lead to yield increases in crops. Certain commercial plant growth regulators tend to increase the susceptibility of plants to fungal disease. The use as a plant growth regulator of a compound having fungicidal properties has clear advantages.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating agricultural compositions may be used. Compositions according to the invention generally contain from 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example, a composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated caster oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 and 75% w of active ingredient and usually contain, in addition to solid inert carrier, 3—10% w of a dispersing agent and, where necessary, 0—10% w of stabiliser(s) and/or other additives such as penetrants or stickers. Dusts are usually formulated as a dust concentrate having similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—25% w active ingredient and 0—10% of additives such as stabilisers, slow release modifiers and binding agents. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

Compositions according to the invention may also contain other biologically active compounds, e.g., insecticides, fungicides or plant growth regulants.

The invention is further illustrated by the following Examples. In each of these examples the structure of the product was confirmed by NMR analysis.

Examples I to IV illustrate preparation of the intermediate compounds of formulae II and IV; Examples V to XXI illustrate the preparation of compounds of the invention. Examples XXII and XXIII illustrate biological activity.

Example I

Preparation of 1-(4-fluorophenyl)-2-(2-methyl-2-propylthio)ethanone

2-Methylpropane-2-thiol (9.02 g, 0.1 mole) was dissolved in dry 1,2-dimethoxyethane (60 ml). Sodium hydride (0.1 mole) was added and the mixture stirred, under nitrogen, for 16 hours at 20°C.

2-bromo-1-(4-fluorophenyl)ethanone (21.7 g; 0.1 mole) dissolved in 1,2-dimethoxyethane was added dropwise over 30 minutes and the mixture stirred for a further 4 hours.

Filtration of the sodium bromide followed by evaporation of the filtrate yielded a red oil (24.3 g). Purification by silica gel chromatography gave the product as a yellow oil (16.0 g; 72% yield).

| | | | | |
|---|---|---|---|---|
| ANALYSIS | Calc. | 63.7% C; | 6.6% H | (for $C_{12}H_{15}SOF$) |
| | Found | 63.5% C; | 6.6% H | |

Example II

Preparation of 1-(4-chlorophenyl)-2-(1-propylthio)ethanone

The sodium salt of 1-propanethiol was prepared by dissolving sodium (2.3 g; 0.1 mole) in methanol (100 ml) and adding the thiol (7.6 g; 0.1 mole) and evaporating to dryness.

The salt was immediately suspended in dry 1,2-dimethylethane (100 ml) and a solution of 2-bromo-1-(4-chlorophenyl)ethanone (23.4 g; 0.1 mole) in 1,2-dimethoxyethane (75 ml) was added dropwise with stirring, at 20°C.

When reaction was complete, the sodium bromide was filtered off and the solvent evaporated. The product was isolated from the residue by chromatography to give a pale brown oil (12.0 g, 52% isolated yield).

| | | | | |
|---|---|---|---|---|
| ANALYSIS | Calc. | 57.8% C; | 5.7% H | (for $C_{11}H_{13}$ SOCl) |
| | Found | 57.3% C; | 5.7% H | |

Example III

Preparation of 2-bromo-1-(4-chlorophenyl)-2-(2-methylpropylthio)ethanone

1-(4-Chlorophenyl)-2-(2-methyl-2-propylthio)ethanone (48.5 g; 0.2 mole) was dissolved in carbon tetrachloride (500 ml). A solution of bromine (32.0 g; 0.2 mole) in carbon tetrachloride (150 ml) was added dropwise with stirring at 20°C. A 300 watt lamp was shone onto the glass reaction vessel for a short time to initiate halogenation. Decolouration of the bromine was then immediate.

At the end of the reaction, the solvent and dissolved gases were evaporated *in vacuo*, and the residue was redissolved in ether and washed with saturated sodium bicarbonate solution. The ether solution was then washed once with water, dried over anhydrous magnesium sulphate and the ether removed *in vacuo* to yield a pale brown oil (55.0 g; 85% yield).

The NMR spectrum was consistent with the required structure.

Example IV

Preparation of 2-bromo-1-(4-chlorophenyl)-2-(phenylthio)ethanone

1-(4-chlorophenyl)-2-(phenylthio)ethanone (10.0 g, 0.038 mole) was dissolved in carbon tetrachloride (80 ml). A solution of bromine (6.1 g; 0.038 mole) in carbon tetrachloride (10 ml) was added dropwise with stirring at 20°C, (decoloration was almost immediate) and then the solvent and dissolved gases were evaporated *in vacuo* to give a pale yellow oil (13.0 g; 100%).

The oil solidified on standing to give pale yellow crystals of m.p. 54—55°C.

| | | | | |
|---|---|---|---|---|
| ANALYSIS | Calc. | 49.2% C; | 2.9% H | (for $C_{14}H_{10}$SOBrCl) |
| | Found | 48.7% C; | 2.8% H | |

Example V

Preparation of 1-(4-chlorophenyl)-2-(2-methyl-2-propylthio)-2-(1,2,4-triazolyl)ethanone

2-bromo-2-(2-methyl-2-propylthio)-1-(4-chlorophenyl)ethanone (55.0 g; 0.17 mole) was dissolved in dry acetonitrile (275 ml). 1,2,4-Triazole (46.9 g; 0.68 mole) was added and the mixture refluxed for $3\frac{1}{2}$ hours. The solvent was removed *in vacuo* and the residue treated with water (250 ml) and methylene chloride

(250 ml). The organic layer was separated and the aqueous layer washed with two portions of methylene chloride (150 ml each). The combined organic extracts were dried (sodium sulphate) and the solvent evaporated.

Purification of the crude product by silica gel chromatography afforded the pure material as a pale yellow solid of m.p. 97—99° (17.0 g, 32% yield)

| | | | | | |
|---|---|---|---|---|---|
| ANALYSIS | Calc. | 54.3% C; | 5.2% H; | 13.6% N | (for $C_{14}H_{16}N_3SOCl$) |
| | Found | 54.1% C; | 5.2% H; | 13.4% N | |

Example VI

Preparation of 1-(4-tolyl)-2-(2-methyl-2-propylthio)-2-(1,2,4-triazolyl)ethanone

2-Bromo-2-(2-methyl-2-propylthio)-1-(4-tolyl)ethanone (13.1 g; 0.041 mole) was dissolved in dry acetonitrile (100 ml). 1,2,4-Triazole (11.3 g; 0.164 mole) was added and the mixture refluxed for 16 hours.

The reaction mixture was worked up and the product isolated in a similar manner to the previous example. This gave a pale yellow-brown solid of m.p. 97—99°C (5.0 g; 42% yield).

| | | | | | |
|---|---|---|---|---|---|
| ANALYSIS | Calc. | 62.4% C; | 6.6% H; | 14.5% N | (for $C_{15}H_{19}N_3SO$) |
| | Found | 62.7% C; | 6.6% H; | 14.6% N | |

Example VII

Preparation of 1-(4-chlorophenyl)2-(2-methyl-2-propylthio)-2-(1,2,4,-triazolyl)ethanol

The compound of Example V (2.0 g, 0.0065 mole) was dissolved in absolute ethanol (50 $cm^3$) and sodium borohydride (0.49 g, 0.013 mole) was added in portions. The stirred mixture was warmed to 40°C for ½ hour and then allowed to cool and stand at room temperature for 20 hours.

The solvent was then evaporated off and the residue treated with water (60 $cm^3$) and extracted with dichloromethane. The organic extract was dried over sodium sulphate and evaporated to dryness.

Recrystallisation of the product from aqueous ethanol gave 1.5 g of the desired product as white crystals, melting point 160—162°C.

| | | | | | |
|---|---|---|---|---|---|
| ANALYSIS | Calc. | 54.0% C; | 5.8% H; | 13.5% N | (for $C_{14}H_{18}N_3SOCl$) |
| | Found | 53.9% C; | 5.8% H; | 13.4% N | |

Examples VIII to XIX

In Examples VIII to XIX the compounds were prepared by methods similar to those of the above Examples. The compounds and their melting points and chemical analyses are shown in Table I below.

TABLE I

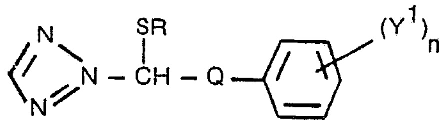

| Example No. | $(Y^1)_n$ | R | Q | M.p.(°C) | ANALYSIS | | | |
|---|---|---|---|---|---|---|---|---|
| VIII | 4-Cl | $-(CH_2)_2CH_3$ | C=O | Oil | CALC. | 52.9%C; | 4.7%H; | 14.2%N |
| | | | | | FOUND | 52.6%C; | 4.8%H; | 14.0%N |
| IX | 4-Cl | Ph | C=O | 102—103 | CALC. | 58.2%C; | 3.6%H; | 12.7%N |
| | | | | | FOUND | 57.8%C; | 3.6%H; | 12.7%N |
| X | 2,4-$Cl_2$ | $-C(CH_3)_3$ | C=O | Oil | CALC. | 48.8%C; | 4.4%H; | 12.2%N |
| | | | | | FOUND | 48.2%C; | 4.4%H; | 11.9%N |
| XI | 2,4-$(CH_3)_2$ | $-C(CH_3)_3$ | C=O | Oil | CALC. | 63.4%C; | 6.9%H; | 13.9%N |
| | | | | | FOUND | 61.7%C; | 7.0%H; | 13.2%N |
| XII | 3,4-$Cl_2$ | $-C(CH_3)_3$ | C=O | 114—115 | CALC. | 48.8%C; | 4.4%H; | 12.2%N |
| | | | | | FOUND | 48.8%C; | 4.5%H; | 12.0%N |

TABLE I (continued)

| Example No. | $(Y^1)_n$ | R | Q | M.p.(°C) | ANALYSIS | | | |
|---|---|---|---|---|---|---|---|---|
| XIII | 4-F | $-C(CH_3)_3$ | C=O | 72—74 | CALC. | 57.3%C; | 5.5%H; | 14.3%N |
| | | | | | FOUND | 56.9%C; | 5.8%H; | 14.3%N |
| XIV | $3-CH_3$; 4-Cl | $-C(CH_3)_3$ | C=O | 92—93 | CALC. | 55.7%C; | 5.6%H; | 13.0%N |
| | | | | | FOUND | 55.8%C; | 5.7%H; | 13.0%N |
| XV | $4-NO_2$ | $-C(CH_3)_3$ | C=O | 136—137 | CALC. | 52.5%C; | 5.0%H; | 17.5%N |
| | | | | | FOUND | 52.6%C; | 5.0%H; | 17.4%N |
| XVI | 4-CN | $-C(CH_3)_3$ | C=O | 104—106 | CALC. | 60.0%C; | 5.3%H; | 18.7%N |
| | | | | | FOUND | 59.9%C; | 5.0%H; | 18.2%N |
| XVII | $4-CH_3$ | $-C(CH_3)_3$ | CH.OH | 158—161 | CALC. | 62.0%C; | 7.2%H; | 14.4%N |
| | | | | | FOUND | 62.4%C; | 7.3%H; | 14.6%N |
| XVIII | 4-F | $-C(CH_3)_3$ | CH.OH | 136—138 | CALC. | 57.0%C; | 6.1%H; | 14.2%N |
| | | | | | FOUND | 57.0%C; | 5.9%H; | 14.2%N |
| XIX | $3,4-Cl_2$ | $-C(CH_3)_3$ | CH.OH | 150—151 | CALC. | 48.5%C; | 4.9%H; | 12.1%N |
| | | | | | FOUND | 47.7%C; | 5.0%H; | 12.0%N |

## Example XX

Preparation of the hydrochloride salt of the compound of Example V

0.9 g of the compound obtained as in Example V were dissolved in dry diethyl ether (100 $cm^3$) and dry hydrogen chloride gas was passed into the stirred solution. A yellow precipitate was formed. When precipitation was complete, the solid was filtered off and washed with diethyl ether, whereupon the solid turned into an oil. This oil was dissolved in chloroform and the solution evaporated to give a sticky yellow solid, which was washed with petroleum ether and dried to give 0.4 g of the desired product as a yellow solid, melting point 133—136°C.

## Example XXI

Preparation of the copper II Chloride complex of the compound of Example V

The compound obtained as in Example V (1.0 g, 0.0032 mol) was dissolved in ethanol (10 $cm^3$) and a solution of $CuCl_2$ (0.22 g, 0.0016 mol) in ethanol (5 $cm^3$) was added. The mixture was stirred at room temperature for 1½ hours, and the resulting blue precipitate was filtered off and washed first with ethanol and then with diethyl ether. After drying, 1.1 g of the desired product, a complex of 2 moles of the compound of Example V per mole of $CuCl_2$ were obtained as a light blue solid, melting point 184—185°C (with decomposition).

## Example XXII

The fungicidal activity of compounds of the invention is illustrated by the following tests which were carried out.

(a) *Activity against apple powdery mildew (Podosphaera leuco tricha; P.1)*

The test is a direct anti-sporulant one using a foliar spray. The upper surfaces of leaves of whole apple seedlings are inoculated by spraying with an aqueous suspension containing $10^5$ conidia/ml 2 days prior to treatment with the test compound. The inoculated plants are immediately dried and kept at glasshouse ambient temperatures and humidity prior to treatment. The plants are sprayed at a dosage of 1 kilogram of active material per hectare using a track sprayer. After drying the plants are returned to a compartment at ambient temperature and humidity for up to 9 days, followed by assessment. Assessment is based on the percentage of the leaf area covered by sporulation compared with that on leaves of control plants.

(b) *Activity against barley powdery mildew (Erysiphe graminis; Eg.)*

The test measures the direct anti-sporulant activity of compounds applied as a foliar spray. For each compound about 40 barley seedlings were grown to the one-leaf stage in a plastic pot of sterile potting compost. Inoculation was effected by dusting the leaves with conidia of Erysiphe graminis, spp. hordei. 24 hours after inoculation the seedlings were sprayed with a solution of the compound in a mixture of acetone (50%), surfactant (0.04%) and water using a track sprayer. The rate of application was equivalent to 1 kg of active material per hectare. First assessment of disease was made 5 days after treatment, when the overall level of sporulation on the treated plants were compared with that on control plants.

The extent of disease control achieved in these tests is expressed as a control rating; greater than 80% disease control is given the rating 2, and control of between 50 and 80% is given the rating 1.

The results of these tests are shown in Table II below, the code given in the table for each test corresponding with those given above.

TABLE II

| Compound of Example No. | Test P.1. | Test E.g. |
|---|---|---|
| V | 2 | 2 |
| VI | 2 | 2 |
| VII | 2 | 1 |
| VIII | 2 | 1 |
| IX | 2 | 2 |
| X | 0 | 2 |
| XI | 2 | 2 |
| XII | 1 | 2 |
| XIII | 2 | 2 |
| XIV | 2 | 2 |
| XV | 0 | 1 |
| XVI | * | 2 |
| XVII | 0 | 2 |
| XVIII | 1 | 2 |
| XIX | 1 | 2 |

* Not tested.

Example XXIII

In this example formulations of the compound of Example VII were tested for plant growth regulating activity on a representative range of plants: maize, *Zea mays*; rice, *Oryza sativa*; barnyard grass, *Echinochloa crusgalli*; oat *Avena sativa*; linseed, *Linum usitatissimum*; mustard, *Sinapis alba*; sugar beet, *Beta vulgaris* and soya bean, *Glycine max*.

The tests carried out were soil drench, foliar spray and pre-emergence tests. In the soil drench, the soil in which young seedlings are growing is drenched with the test solution; in the foliar test, the foliage of young seedlings is sprayed with a formulation containing the test compound; in the preemergence test soil planted with seeds of the various plant species was treated with the test compound. The soil used in the tests was a prepared horticultural loam.

The formulations used in the tests were prepared by diluting with water, solutions of the test compound in acetone containing 0.4% by weight of an alkylphenol/ethylene oxide condensate available under the trade mark TRITON X—155. In the foliar spray and pre-emergence tests, the acetone solution was diluted with an equal volume of water and the resulting formulations applied at a dosage level corresponding to 5 kg of active material per hectare in a volume equivalent to 650 litres per hectare. In the soil drench tests, one volume of the acetone solution was diluted with 155 volumes of water and the resulting formulation applied at a dosage level equivalent to 10 kg of active material per hectare in a volume equivalent to approximately 3,000 litres per hectare.

Untreated seedling plants or seeds were used as controls.

Observations were made over a period of 11 days after spraying, and a marked depression of growth (i.e. a reduction in stem height) was noted in respect of maize, rice, black grass, oat, linseed, mustard, sugar beet and soya bean in the drench tests, in respect of black grass, linseed, mustard sugar beet and soya bean

in the foliar tests, and oats, linseed, sugar beet and soya bean in the preemergence tests. In all these cases, symptoms of hyperchromism — i.e., the production of very dark green leaves — were noted.

**Claims for the Contracting States: BE CH DE FR GB IT LI LU NL SE**

1. A fungicidal and/or plant growth regulating composition, which comprises a carrier and, in association therewith, a compound of the general formula

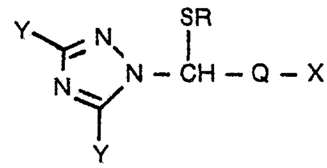

(I)

or an acid addition salt or metal salt complex thereof, wherein X represents a phenyl group which is unsubstituted or substituted by up to 3 of the same or different substituents selected from halogen atoms and cyano, nitro, methylenedioxy, $C_{1-6}$ alkylsulphonyl, $C_{1-6}$ alkyl, $C_{1-6}$ haloalkyl, $C_{2-6}$ alkenyl, $C_{2-6}$ alkynyl, $C_{1-6}$ alkoxy, $C_{1-6}$ haloalkoxy, $C_{1-6}$ alkylthio, ($C_{1-6}$ alkoxy)carbonyl, phenyl and phenoxy groups; each Y independently represents a hydrogen or halogen atom or a $C_{1-6}$ alkyl group; Q represents a —CO—, —CS—, —CH.OH— or —CH.SH— group; and R represents a hydrogen atom, an alkyl group having up to 12 carbon atoms, which is unsubstituted or substituted by one or more substituents selected from halogen atoms, $C_{1-6}$ alkoxy, $C_{1-6}$ alkylthio, cyano, carboxy, ($C_{1-6}$ alkoxy)carbonyl, hydroxy, amino, $C_{3-7}$ cycloalkyl, phenyl and phenoxy groups, a $C_{3-7}$ cycloalkyl group which is unsubstituted or substituted by one or more substituents selected from halogen atoms and $C_{1-6}$ alkyl, $C_{1-6}$ alkoxy and $C_{1-6}$ alkylthio groups, or a phenyl group which is unsubstituted or substituted by up to 3 of the same or different substituents selected from halogen atoms and cyano, nitro, methylenedioxy, $C_{1-6}$ alkylsulphonyl, $C_{1-6}$ alkyl, $C_{1-6}$ haloalkyl, $C_{2-6}$ alkenyl, $C_{2-6}$ alkynyl, $C_{1-6}$ alkoxy, $C_{1-6}$ haloalkoxy, $C_{1-6}$ alkylthio, ($C_{1-6}$ alkoxy)carbonyl, phenyl and phenoxy groups.

2. A composition as claimed in claim 1, which comprises at least two carriers, at least one of which is a surface-active agent.

3. A composition as claimed in claim 1 or 2, in which the phenyl group X is unsubstituted or substituted by up to 3 of the same or different substituents selected from halogen atoms and methyl, methoxy, trifluoromethyl, trifluoromethoxy, methylthio, cyano and nitro groups.

4. A composition as claimed in claim 3, in which X represents a mono or di-halophenyl group.

5. A composition as claimed in any one of claims 1 to 4, in which both Y's represent hydrogen atoms.

6. A composition as claimed in any one of claims 1 to 5, in which Q represents a —CO— or —CH.OH— group.

7. A composition as claimed in any one of claims 1 to 6, in which R represents an alkyl group having up to 12 carbon atoms, which is unsubstituted or substituted by one or more halogen atoms, or a phenyl group which is unsubstituted or substituted by up to 3 of the substituents defined in claim 3.

8. A composition as claimed in claim 7, in which R represents an unsubstituted alkyl group having up to 6 carbon atoms.

9. A compound of formula I as defined in any one of Claims 1 and 3 to 8, provided that when X represents a phenyl group which is unsubstituted or substituted exclusively by one or more substituents selected from halogen atoms and cyano, $C_{1-6}$ alkyl, $C_{1-4}$ alkoxy, phenyl and phenoxy groups, each Y represents a hydrogen atom and R represents a phenyl group which is unsubstituted or substituted exclusively by one or more substituents selected from halogen atoms and $C_{1-4}$ alkyl, $C_{1-4}$ alkoxy, cyano, phenyl and phenoxy groups, Q represents a —CO—, —CS—, or —CH.SH— group.

10. A process for the preparation of a compound as claimed in claim 9, which comprises reacting a 2-halo-1-phenylethane derivative of formula:

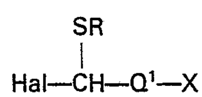

(II)

wherein X and R are as defined in Claim 9, $Q^1$ is a —CO— or —CS— group, and Hal is a halogen atom, with a triazole derivative of formula:—

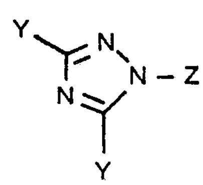

(III)

wherein Y is as defined in Claim 9 and Z represents a hydrogen atom or one equivalent of an alkali or alkaline earth metal; and if desired, converting the resulting compound of formula I into another compound of formula I.

11. The use as a fungicide and/or plant growth regulant of a compound of formula I as defined in any one of claims 1 and 3 to 9 or a composition as claimed in any one of claims 1 to 8.

12. A method of combating fungus and/or regulating plant growth at a locus, which comprises applying to the locus a compound of formula I as defined in any one of claims 1 and 3 to 9 or a composition as claimed in any one of claims 1 to 8.

**Claims for the Contracting State: AT**

1. A fungicidal and/or plant growth regulating composition, which comprises a carrier and, in association therewith, a compound of the general formula

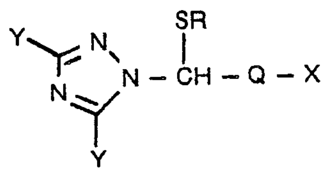

(I)

or an acid addition salt or metal salt complex thereof, wherein X represents a phenyl group which is unsubstituted or substituted by up to 3 of the same or different substituents selected from halogen atoms and cyano, nitro, methylenedioxy, $C_{1-6}$ alkylsulphonyl, $C_{1-6}$ alkyl, $C_{1-6}$ haloalkyl, $C_{2-6}$ alkenyl, $C_{2-6}$ alkynyl, $C_{1-6}$ alkoxy, $C_{1-6}$ haloalkoxy, $C_{1-6}$ alkylthio, ($C_{1-6}$ alkoxy)carbonyl, phenyl and phenoxy groups; each Y independently represents a hydrogen or halogen atom or a $C_{1-6}$ alkyl group; Q represents a —CO—, —CS—, —CH.OH— or —CH.SH— group; and R represents a hydrogen atom, an alkyl group having up to 12 carbon atoms, which is unsubstituted or substituted by one or more substituents selected from halogen atoms, $C_{1-6}$ alkoxy, $C_{1-6}$ alkylthio, cyano, carboxy, ($C_{1-6}$ alkoxy)carbonyl, hydroxy, amino, $C_{3-7}$ cycloalkyl, phenyl and phenoxy groups, a $C_{3-7}$ cycloalkyl group which is unsubstituted or substituted by one or more substituents selected from halogen atoms and $C_{1-6}$ alkyl, $C_{1-6}$ alkoxy and $C_{1-6}$ alkylthio groups, or a phenyl group which is unsubstituted or substituted by up to 3 of the same or different substituents selected from halogen atoms and cyano, nitro, methylenedioxy, $C_{1-6}$ alkylsulphonyl, $C_{1-6}$ alkyl, $C_{1-6}$ haloalkyl, $C_{2-6}$ alkenyl, $C_{2-6}$ alkynyl, $C_{1-6}$ alkoxy, $C_{1-6}$ haloalkoxy, $C_{1-6}$ alkylthio, ($C_{1-6}$ alkoxy)carbonyl, phenyl and phenoxy groups.

2. A composition as claimed in claim 1, which comprises at least two carriers, at least one of which is a surface-active agent.

3. A composition as claimed in claim 1 or 2, in which the phenyl group X is unsubstituted or substituted by up to 3 of the same or different substituents selected from halogen atoms and methyl, methoxy, trifluoromethyl, trifluoromethoxy, methylthio, cyano and nitro groups.

4. A composition as claimed in claim 3, in which X represents a mono or di-halophenyl group.

5. A composition as claimed in any one of claims 1 to 4, in which both Y's represent hydrogen atoms.

6. A composition as claimed in any one of claims 1 to 5, in which Q represents a —CO— or —CH.OH— group.

7. A composition as claimed in any one of claims 1 to 6, in which R represents an alkyl group having up to 12 carbon atoms, which is unsubstituted or substituted by one or more halogen atoms, or a phenyl group which is unsubstituted or substituted by up to 3 of the substituents defined in claim 3.

8. A composition as claimed in claim 7, in which R represents an unsubstituted alkyl group having up to 6 carbon atoms.

9. A process for the preparation of a compound as defined in claim 1, which comprises reacting a 2-halo-1-phenylethane derivative of formula:

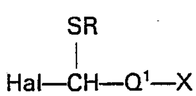

(II)

wherein X and R are as defined in Claim 1, $Q^1$ is a —CO— or —CS— group, and Hal is a halogen atom, with a triazole derivative of formula:—

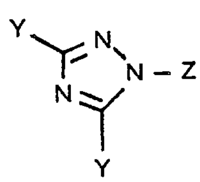

(III)

wherein Y is as defined in Claim 1 and Z represents a hydrogen atom or one equivalent of an alkali or alkaline earth metal; and if desired, converting the resulting compound of formula I into another compound of formula I.

10. The use as a fungicide and/or plant growth regulant of a compound of formula I as defined in any one of claims 1 and 3 to 8 or a composition as claimed in any one of claims 1 to 8.

11. A method of combating fungus and/or regulating plant growth at a locus, which comprises applying to the locus a compound of formula I as defined in any one of claims 1 and 3 to 8 or a composition as claimed in any one of claims 1 to 8.

**Patentansprüche für die Vertragsstaaten: BE CH DE FR GB IT LI LU NL SE**

1. Fungicide und/oder das Pflanzenwachstum regulierende Zusammensetzung, welche einen Träger und in Verbindung damit eine Verbindung der allgemeinen Formel

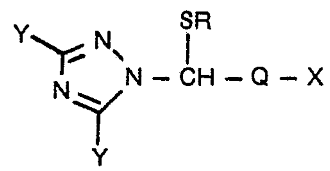

(I)

oder ein Säureadditionssalz oder einen Metallsalzkomplex hievon umfaßt, worin X eine Phenylgruppe bedeutet, die unsubstituiert oder durch bis zu drei gleiche oder verschiedene Substituenten, ausgewählt unter Halogenatomen und Cyano, Nitro, Methylendioxy, $C_{1-6}$-Alkylsulfonyl, $C_{1-6}$-Alkyl, $C_{1-6}$-Halogenalkyl, $C_{2-6}$-Alkenyl, $C_{2-6}$-Alkinyl, $C_{1-6}$-Alkoxy, $C_{1-6}$-Halogenalkoxy, $C_{1-6}$-Alkylthio, ($C_{1-6}$-Alkoxy)carbonyl, Phenyl- und Phenoxygruppen, substituiert ist; jedes Y unabhängig voneinander ein Wasserstoff- oder Halogenatom oder eine $C_{1-6}$-Alkylgruppe darstellt; Q eine —CO—, —CS—, —CH.OH— oder —CH.SH-Gruppe darstellt; und R ein Wasserstoffatom, eine Alkylgruppe mit bis zu 12 Kohlenstoffatomen, welche unsubstituiert oder durch einen oder mehrere Substituenten, ausgewählt unter Halogenatomen, $C_{1-6}$-Alkoxy, $C_{1-6}$-Alkylthio, Cyano, Carboxy, ($C_{1-6}$-Alkoxy)carbonyl, Hydroxy, Amino, $C_{3-7}$-Cycloalkyl, Phenyl- und Phenoxygruppen, substituiert ist, eine $C_{3-7}$-Cycloalkylgruppe, welche unsubstituiert oder durch einen oder mehrere Substituenten, ausgewählt unter Halogenatomen und $C_{1-6}$-Alkyl, $C_{1-6}$-Alkoxy und $C_{1-6}$-Alkylthiogruppen, substituiert ist, oder eine Phenylgruppe bedeutet, welche unsubstituiert oder durch bis zu drei gleiche oder verschiedene Substituenten, ausgewählt unter Halogenatomen und Cyano, Nitro, Methylendioxy, $C_{1-6}$-Alkylsulfonyl, $C_{1-6}$-Alkyl, $C_{1-6}$-Halogenalkyl, $C_{2-6}$-Alkenyl, $C_{2-6}$-Alkinyl, $C_{1-6}$-Alkoxy, $C_{1-6}$-Halogenalkoxy, $C_{1-6}$-Alkylthio, ($C_{1-6}$-Alkoxy)carbonyl, Phenyl- und Phenoxygruppen, substituiert ist.

2. Zusammensetzung nach Anspruch 1, welche wenigstens zwei Träger umfaßt, von denen wenigstens einer ein oberflächenaktives Mittel ist.

3. Zusammensetzung nach Anspruch 1 oder 2, worin die Phenylgruppe X unsubstituiert oder durch bis zu drei gleiche oder verschiedene Substituenten, ausgewählt unter Halogenatomen, Methyl, Methoxy, Trifluormethyl, Trifluormethoxy, Methylthio, Cyano- und Nitrogruppen, substituiert ist.

4. Zusammensetzung nach Anspruch 3, worin X eine Mono- oder Dihalogenphenylgruppe bedeutet.

5. Zusammensetzung nach einem der Ansprüche 1 bis 4, worin beide Y Wasserstoffatome bedeuten.

6. Zusammensetzung nach einem der Ansprüche 1 bis 5, worin Q eine —CO— oder —CH.OH-Gruppe bedeutet.

7. Zusammensetzung nach einem der Ansprüche 1 bis 6, worin R eine Alkylgruppe mit bis zu 12 Kohlenstoffatomen, welche unsubstituiert oder durch ein oder mehrere Halogenatome substituiert ist, oder eine Phenylgruppe bedeutet, welche unsubstituiert oder durch bis zu 3 der in Anspruch 3 definierten Substituenten substituiert ist.

8. Zusammensetzung nach Anspruch 7, worin R eine unsubstituierte Alkylgruppe mit bis zu 6 Kohlenstoffatomen bedeutet.

9. Verbindung der Formel I, wie in einem der Ansprüche 1 und 3 bis 8 definiert, mit der Maßgabe, daß dann, wenn X eine Phenylgruppe darstellt, die unsubstituiert oder ausschließlich durch einen oder mehrere Substituenten, ausgewählt unter Halogenatomen und Cyano, $C_{1-6}$-Alkyl, $C_{1-4}$-Alkoxy, Phenyl- und Phenoxygruppen, substituiert ist, jedes Y ein Wasserstoffatom darstellt und R eine Phenylgruppe darstellt, die unsubstituiert oder ausschließlich durch einen mehrere Substituenten, ausgewählt unter Halogenatomen und $C_{1-4}$-Alkyl, $C_{1-4}$-Alkoxy, Cyano, Phenyl- und Phenoxygruppen, substituiert ist, Q eine —CO—, —CS— oder —CH.SH-Gruppe bedeutet.

10. Verfahren zur Herstellung einer Verbindung, wie in Anspruch 9 beansprucht, welches ein Umsetzen eines 2-Halogen-1-phenylethan-Derivats der Formel:

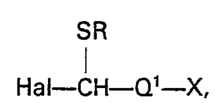

(II)

worin X und R wie in Anspruch 9 definiert sind, $Q^1$ eine —CO— oder CS-Gruppe ist und Hal ein Halogenatom bedeutet, mit einem Triazol-Derivat der Formel:

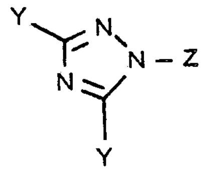

(III)

worin Y wie in Anspruch 9 definiert ist und Z ein Wasserstoffatom oder ein Äquivalent eines Alkali- oder Erdalkalimetalles bedeutet; und gewünschtenfalls ein Überführen der erhaltenen Verbindung der Formel I in eine andere Verbindung der Formel I umfaßt.

11. Verwendung einer Verbindung der Formel I, wie in einem der Ansprüche 1 und 3 bis 9 definiert, oder einer Zusammensetzung, wie in einem der Ansprüche 1 bis 8 beansprucht, als Fungicid und/oder Pflanzenwachstum-regulierendes Mittel.

12. Verfahren zur Bekämpfung von Pilzen und/oder zur Regulierung des Pflanzenwachstums an einem Ort, welches ein Aufbringen einer Verbindung der Formel I, wie in einem der Ansprüche 1 und 3 bis 9 definiert, oder einer Zusammensetzung, wie in einem der Ansprüche 1 bis 8 beansprucht, auf den Ort umfaßt.

**Patentansprüche für den Vertragsstaat: AT**

1. Fungicide und/oder das Pflanzenwachstum regulierende Zusammensetzung, welche einen Träger und in Verbindung damit eine Verbindung der allgemeinen Formel

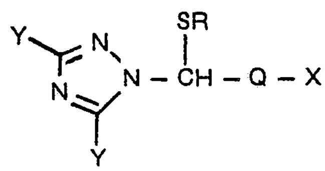

(I)

oder ein Säureadditionssalz oder einen Metallsalzkomplex hievon umfaßt, worin X eine Phenylgruppe bedeutet, die unsubstituiert oder durch bis zu drei gleiche oder verschiedene Substituenten, ausgewählt unter Halogenatomen und Cyano, Nitro, Methylendioxy, $C_{1-6}$-Alkylsulfonyl, $C_{1-6}$-Alkyl, $C_{1-6}$-Halogenalkyl, $C_{2-6}$-Alkenyl, $C_{2-6}$-Alkinyl, $C_{1-6}$-Alkoxy, $C_{1-6}$-Halogenalkoxy, $C_{1-6}$-Alkylthio, ($C_{1-6}$-Alkoxy)carbonyl, Phenyl- und Phenoxygruppen, substituiert ist; jedes Y unabhängig voneinander ein Wasserstoff- oder Halogenatom oder eine $C_{1-6}$-Alkylgruppe darstellt; Q eine —CO—, —CS—, —CH.OH— oder —CH.SH-Gruppe darstellt; und R ein Wasserstoffatom, eine Alkylgruppe mit bis zu 12 Kohlenstoffatomen, welche unsubstituiert oder durch einen oder mehrere Substituenten, ausgewählt unter Halogenatomen, $C_{1-6}$-Alkoxy, $C_{1-6}$-Alkylthio, Cyano, Carboxy, ($C_{1-6}$-Alkoxy)carbonyl, Hydroxy, Amino, $C_{3-7}$-Cycloalkyl, Phenyl- und Phenoxygruppen, substituiert ist, eine $C_{3-7}$-Cycloalkylgruppe, welche unsubstituiert oder durch einen oder mehrere Substituenten, ausgewählt unter Halogenatomen und $C_{1-6}$-Alkyl, $C_{1-6}$-Alkoxy und $C_{1-6}$-Alkylthiogruppen, substituiert ist, oder eine Phenylgruppe bedeutet, welche unsubstituiert oder durch bis zu drei gleiche oder verschiedene Substituenten, ausgewählt unter Halogenatomen und Cyano, Nitro, Methylendioxy, $C_{1-6}$-Alkylsulfonyl, $C_{1-6}$-Alkyl, $C_{1-6}$-Halogenalkyl, $C_{2-6}$-Alkenyl, $C_{2-6}$-Alkinyl, $C_{1-6}$-Alkoxy, $C_{1-6}$-Halogenalkoxy, $C_{1-6}$-Alkylthio, ($C_{1-6}$-Alkoxy)carbonyl, Phenyl- und Phenoxygruppen, substituiert ist.

2. Zusammensetzung nach Anspruch 1, welche wenigstens zwei Träger umfaßt, von denen wenigstens einer ein oberflächenaktives Mittel ist.

3. Zusammensetzung nach Anspruch 1 oder 2, worin die Phenylgruppe X unsubstituiert oder durch bis zu drei gleiche oder verschiedene Substituenten, ausgewählt unter Halogenatomen, Methyl, Methoxy, Trifluormethyl, Trifluormethoxy, Methylthio, Cyano- und Nitrogruppen, substituiert ist.

4. Zusammensetzung nach Anspruch 3, worin X eine Mono- oder Dihalogenphenylgruppe bedeutet.

5. Zusammensetzung nach einem der Ansprüche 1 bis 4, worin beide Y Wasserstoffatome bedeuten.

6. Zusammensetzung nach einem der Ansprüche 1 bis 5, worin Q eine —CO— oder —CH.OH-Gruppe bedeutet.

7. Zusammensetzung nach einem der Ansprüche 1 bis 6, worin R eine Alkylgruppe mit bis zu 12 Kohlenstoffatomen, welche unsubstituiert oder durch ein oder mehrere Halogenatome substituiert ist, oder eine Phenylgruppe bedeutet, welche unsubstituiert oder durch bis zu 3 der in Anspruch 3 definierten Substituenten substituiert ist.

8. Zusammensetzung nach Anspruch 7, worin R eine unsubstituierte Alkylgruppe mit bis zu 6 Kohlenstoffatomen bedeutet.

9. Verfahren zur Herstellung einer Verbindung, wie in Anspruch 1 definiert, welches ein Umsetzen eines 2-Halogen-1-phenylethan-Derivats der Formel:

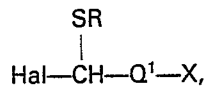

(II)

worin X und R wie in Anspruch 1 definiert sind, Q' eine —CO— oder CS-Gruppe bedeutet und Hal ein Halogenatom darstellt, mit einem Triazol-Derivat der Formel:

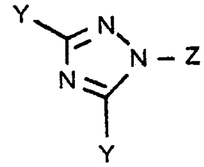

(III)

worin Y wie in Anspruch 1 definiert ist und Z ein Wasserstoffatom oder ein Äquivalent eines Alkali- oder Erdalkalimetalles bedeutet; und gewünschtenfalls ein Überführen der erhaltenen Verbindung der Formel I in eine andere Verbindung der Formel I umfaßt.

10. Verwendung einer Verbindung der Formel I, wie in einem der Ansprüche 1 und 3 bis 8 definiert, oder einer Zusammensetzung, wie in einem der Ansprüche 1 bis 8 definiert, als Fungicid und/oder Pflanzenwachstum-regulierendes Mittel.

11. Verfahren zur Bekämpfung von Pilzen und/oder zur Regulierung des Pflanzenwachstums an einem Ort, welches ein Aufbringen einer Verbindung der Formel I, wie in einem der Ansprüche 1 und 3 bis 8 definiert, oder einer Zusammensetzung, wie in einem der Ansprüche 1 bis 8 beansprucht, auf den Ort umfaßt.

**Revendications pour les Etats contractants: BE CH DE FR GB IT LU NL SE LI**

1. Une composition fongicide et/ou pour réglage de la croissance de plantes, qui comprend un véhicule et, en association avec lui, un composé de la formule générale:

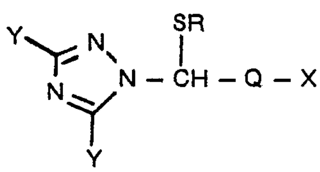

(I)

ou un sel d'addition d'acide ou un complexe de sel de métal d'un tel composé, où X représente un groupe phényle qui est non-substitué ou substitué par jusqu'à 3 substituants identiques ou différents choisis parmi des atomes d'halogènes et des groupes cyano, nitro, méthylènedioxy, $C_{1-6}$ alcoylsulfonyle, alcoyle en $C_{1-6}$, haloalcoyle en $C_{1-6}$, alcényle en $C_{2-6}$, alcynyle en $C_{2-6}$, alcoxy en $C_{1-6}$, haloalcoxy en $C_{1-6}$, $C_{1-6}$ alcoylthio, (alcoxy en $C_{1-6}$)carbonyle, phényle et phénoxy; chaque Y indépendamment représente un atome d'hydrogène ou d'halogène ou un groupe alcoyle en $C_{1-6}$; Q représente un groupe —CO—, —CS—, —CH.OH— ou —CH.SH—; et R représente un atome d'hydrogène, un groupe alcoyle ayant jusqu'à 12 atomes de carbone, qui est non-substitué ou substitué par un ou plusieurs substituants choisis parmi des atomes d'halogènes, des groupes alcoxy en $C_{1-6}$, $C_{1-6}$ alcoylthio, cyano, carboxy, (alcoxy en $C_{1-6}$)carbonyle, hydroxy, amino, cycloalcoyle en $C_{3-7}$, phényle et phénoxy, un groupe cycloalcoyle en $C_{3-7}$ qui est non-substitué ou substitué par un ou plusieurs substituants choisis parmi des atomes d'halogènes et des groupes alcoyle en $C_{1-6}$, alcoxy en $C_{1-6}$ et $C_{1-6}$ alcoylthio, ou un groupe phényle qui est non-substitué ou substitué par jusqu'à 3 substituants identiques ou différents choisis parmi des atomes d'halogènes et des groupes cyano, nitro, méthylènedioxy, $C_{1-6}$ alcoylsulfonyle, alcoyle en $C_{1-6}$, haloalcoyle en $C_{1-6}$, alcényle en $C_{2-6}$, alcynyle en $C_{2-6}$, alcoxy en $C_{1-6}$, haloalcoxy en $C_{1-6}$, $C_{1-6}$ alcoylthio, (alcoxy en $C_{1-6}$)carbonyle, phényle et phénoxy.

2. Une composition selon la revendication 1, qui comprend au moins deux véhicules, dont l'un au moins est un agent tensio-actif.

3. Une composition selon la revendication 1 ou 2, dans laquelle le groupe phényle X est non-substitué ou substitué par jusqu'à 3 substituants identiques ou différents choisis parmi des atomes d'halogènes et des groupes méthyle, méthoxy, trifluorométhyle, trifluorométhoxy, méthylthio, cyano et nitro.

4. Une composition selon la revendication 3, dans laquelle X représente un groupe mono ou di-halophényle.

5. Une composition selon l'une quelconque des revendications 1 à 4, dans laquelle les deux Y représentent des atomes d'hydrogène.

6. Une composition selon l'une quelconque des revendications 1 à 5, dans laquelle Q représente un groupe —CO— ou —CH.OH—.

7. Une composition selon l'une quelconque des revendications 1 à 6, dans laquelle R représente un groupe alcoyle ayant jusqu'à 12 atomes de carbone, qui est non-substitué ou substitué par un ou plusieurs atomes d'halogènes, ou un groupe phényle qui est non-substitué ou substitué par jusqu'à 3 des substituants définis dans la revendication 3.

8. Une composition selon la revendication 7, dans laquelle R représente un groupe alcoyle non-substitué ayant jusqu'à 6 atomes de carbone.

9. Un composé de formule I tel que défini dans l'une quelconque des revendications 1 et 3 à 8, pourvu que quand X représente un groupe phényle qui est non-substitué ou substitué exclusivement par un ou plusieurs substituants choisis parmi des atomes d'halogènes et des groupes cyano, alcoyle en $C_{1-6}$, alcoxy en $C_{1-4}$, phényle et phénoxy, chaque Y représente un atome d'hydrogène et R représente un groupe phényle qui est non-substitué ou substitué exclusivement par un ou plusieurs substituants choisis parmi des atomes d'halogènes et des groupes alcoyle en $C_{1-4}$, alcoxy en $C_{1-4}$, cyano, phényle et phénoxy, Q représente un groupe —CO—, —CS— ou —CH.HS—.

10. Un procédé pour la préparation d'un composé tel que défini dans la revendication 9, qui comprend la réaction d'un dérivé de 2-halo-1-phényléthane de formule:

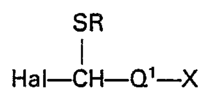

(II)

où X et R sont tels que définis dans la revendication 9, $Q^1$ est un groupe —CO— ou —CS— et Hal est un atome d'halogène, avec un dérivé de triazole de formule:

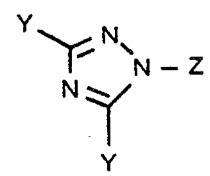

(III)

où Y est tel que défini dans la revendication 9 et Z représente un atome d'hydrogène ou un équivalent d'un métal alcalin ou alcalino-terreux; et, si on le désire, la conversion du composé de formule I résultant en un autre composé de formule I.

1. L'utilisation comme fongicide et/ou agent de réglage de la croissance de plantes d'un composé de formule I tel que défini dans l'une quelconque des revendications 1 et 3 à 9 ou d'une composition selon l'une quelconque des revendications 1 à 8.

12. Un procédé de lutte contre les champignons et/ou de réglage de la croissance de plantes en un lieu, qui comprend l'application à ce lieu d'un composé de formule I tel que défini dans l'une quelconque des revendications 1 et 3 à 9 ou d'une composition selon l'une quelconque des revendications 1 à 8.

**Revendications pour l'Etat contractant: AT**

1. Une composition fongicide et/ou pour réglage de la croissance de plantes, qui comprend un véhicule et, en association avec lui, un composé de la formule générale:

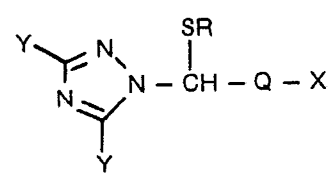

(I)

ou un sel d'addition d'acide ou un complexe de sel de métal d'un tel composé, où X représente un groupe phényle qui est non-substitué ou substitué par jusqu'à 3 substituants identiques ou différents choisis parmi des atomes d'halogènes et des groupes cyano, nitro, méthylènedioxy, $C_{1-6}$ alcoylsulfonyle, alcoyle en $C_{1-6}$, haloalcoyle en $C_{1-6}$, alcényle en $C_{2-6}$, alcynyle en $C_{2-6}$, alcoxy en $C_{1-6}$, haloalcoxy en $C_{1-6}$, $C_{1-6}$ alcoylthio, (alcoxy en $C_{1-6}$)carbonyle, phényle et phénoxy; chaque Y indépendamment représente un atome d'hydrogène ou d'halogène ou un groupe alcoyle en $C_{1-6}$; Q représente un groupe —CO—, —CS—, —CH.OH— ou —CH.SH—; et R représente un atome d'hydrogène, un groupe alcoyle ayant jusqu'à 12 atomes de carbone, qui est non-substitué ou substitué par un ou plusieurs substituants choisis parmi des atomes d'halogènes, des groupes alcoxy en $C_{1-6}$, $C_{1-6}$ alcoylthio, cyano, carboxy, (alcoxy en $C_{1-6}$)carbonyle, hydroxy, amino, cycloalcoyle en $C_{3-7}$, phényle et phénoxy, un groupe cycloalcoyle en $C_{3-7}$ qui est non-substitué ou substitué par un ou plusieurs substituants choisis parmi des atomes d'halogènes

et des groupes alcoyle en $C_{1-6}$, alcoxy en $C_{1-6}$ et $C_{1-6}$ alcoylthio, ou un groupe phényle qui est non-substitué ou substitué par jusqu'à 3 substituants identiques ou différents choisis parmi des atomes d'halogènes et des groupes cyano, nitro, méthylènedioxy, $C_{1-6}$ alcoylsulfonyle, alcoyle en $C_{1-6}$, haloalcoyle en $C_{1-6}$, alcényle en $C_{2-6}$, alcynyle en $C_{2-6}$, alcoxy en $C_{1-6}$, haloalcoxy en $C_{1-6}$, $C_{1-6}$ alcoylthio, (alcoxy en $C_{1-6}$)carbonyle, phényle et phénoxy.

2. Une composition selon la revendication 1, qui comprend au moins deux véhicules, dont l'un au moins est un agent tensio-actif.

3. Une composition selon la revendication 1 ou 2, dans laquelle le groupe phényle X est non-substitué ou substitué par jusqu'à 3 substituants identiques ou différents choisis parmi des atomes d'halogènes et des groupes méthyle, méthoxy, trifluorométhyle, trifluorométhoxy, méthylthio, cyano et nitro.

4. Une composition selon la revendication 3, dans laquelle X représente un groupe mono- ou di-halophényle.

5. Une composition selon l'une quelconque des revendications 1 à 4, dans laquelle les deux Y représentent des atomes d'hydrogène.

6. Une composition selon l'une quelconque des revendications 1 à 5, dans laquelle Q représente un groupe —CO— ou —CH.OH—.

7. Une composition selon l'une quelconque des revendications 1 à 6, dans laquelle R représente un groupe alcoyle ayant jusqu'à 12 atomes de carbone, qui est non-substitué ou substitué par un ou plusieurs atomes d'halogènes, ou un groupe phényle qui est non-substitué ou substitué par jusqu'à 3 des substituants définis dans la revendication 3.

8. Une composition selon la revendication 7, dans laquelle R représente un groupe alcoyle non-substitué ayant jusqu'à 6 atomes de carbone.

9. Un procédé pour la préparation d'un composé tel que défini dans la revendication 1, qui comprend la réaction d'un dérivé de 2-halo-1-phényléthane de formule:

$$\mathrm{Hal{-}\overset{\overset{SR}{|}}{C}H{-}Q^1{-}X} \qquad (II)$$

où X et R sont tels que définis dans la revendication 1, $Q^1$ est un groupe —CO— ou —CS— et Hal est un atome d'halogène avec un dérivé de triazole de formule:

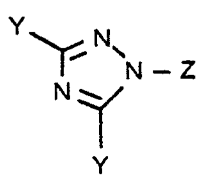

(III)

où Y est tel que défini dans la revendication 1 et Z représente un atome d'hydrogène ou un équivalent d'un métal alcalin ou alcalino-terreux; et, si on le désire, la conversion du composé de formule I résultant en un autre composé de formule I.

10. L'utilisation comme fongicide et/ou agent de réglage de la croissance de plantes d'un composé de formule I tel que défini dans l'une quelconque des revendications 1 et 3 à 8 ou d'une composition selon l'une quelconque des revendications 1 à 8.

11. Un procédé de lutte contre les champignons et/ou de réglage de la croissance de plantes en un lieu, qui comprend l'application à ce lieu d'un composé de formule I tel que défini dans l'une quelconque des revendications 1 et 3 à 8 ou d'une composition selon l'une quelconque des revendications 1 à 8.